(19) Europäisches Patentamt

European Patent Office

Office européen des brevets

(11) Publication number: **0 440 254 A1**

# (12) EUROPEAN PATENT APPLICATION

(21) Application number: **91101365.4**

(22) Date of filing: **01.02.91**

(51) Int. Cl.5: **A61B 1/00**

(30) Priority: **01.02.90 JP 8124/90**

(43) Date of publication of application:
**07.08.91 Bulletin 91/32**

(84) Designated Contracting States:
**DE FR GB**

(71) Applicant: **MACHIDA ENDOSCOPE CO., LTD**
**13-8, Honkomagome 6-chome**
**Bunkyo-ku, Tokyo(JP)**

(72) Inventor: **Chikama, Toshio, c/o Machida**
**Endoscope Co.,Ltd.**
**13-8, Honkomagome 6-chome**
**Bunkyo-ku, Tokyo(JP)**

(74) Representative: **Koch, Günther, Dipl.-Ing.**
**Patentanwälte Wallach, Koch, Dr. Haibach,**
**Feldkamp et al**
**P.O. Box 121120**
**W-8000 München 12(DE)**

(54) **Endoscope cover.**

(57) An endoscope cover comprising a cylindrical rigid cover (6) composed of an elastic material, which is fitted to an endoscope (1) to cover a top end rigid portion (5) of the endoscope.

A transparent window (6b,6c) arranged on a closed face (6a) formed on the top end of the rigid cover to confront at least an observation port and an illuminating port, which are arranged on the top end of the endoscope, and a bag-shaped covering member (7) having a length covering at least an intermediate conduit portion (3) of the endoscope, which is attached to the rear part (2) of the endoscope. The rigid cover (6) is bonded to the top end rigid portion (5) by an adhesive and the covering member (7) is bonded (8) to all or a part of the circumference of the intermediate conduit portion to cover the intermediate conduit portion.

Fig. 2

EP 0 440 254 A1

## ENDOSCOPE COVER

Background of the Invention

The present device relates to a cover for covering the outer periphery of an endoscope when the endoscope is used.

After an endoscope is used in the body cavity of a patient, the endoscope is generally washed and disinfected. However, this washing and disinfecting operation requires much time and labor, and the working efficiency of the endoscope is very low. Furthermore, if this washing and disinfecting operation is not sufficiently performed, no satisfactory washing and disinfecting effect can be attained.

Accordingly, an idea of a disposable oover for covering the outer periphery of an endoscope when the endoscope is used has recently been proposed, and an example of the disposable cover is disclosed in Japanese Unexamined Patent Publication No. 61-179128.

The disclosed technique concerns an endoscope cover comprising a soft cylindrical coverning member composed of a rubber or synthetic resin, which is attached to a rigid cover fitted to cover a top end rigid portion of the endoscope.

According to this endoscope cover, the rigid cover is fitted to the top end rigid portion of the endoscope, a flexible tube of the endoscope is entirely covered with the covering member from the top end side of the flexible tube, the endoscope is inserted in this state into the body cavity of a patient, and after the endoscope is used, the endoscope cover is dismounted and thrown away. When the endoscope is used again, the endoscope is covered with a new endoscope cover and is used in the above-mentioned manner. According to this technique, the endoscope need not be washed and disinfected, and a high sanitary effect is attained and the endoscope can be continuously used.

When the above-mentioned endoscope is inserted into the body cavity for the use, or is bent in the body cavity or repeatedly moved to and fro in the inserting direction, the rigid cover comes off from the top end rigid portion or the covering member gets out of position. Even if the rigid cover does not come off, the field of vision is disturbed by the sippage of the rigid conver.

Furthermore, if the rigid cover is tightly fitted, it is difficult to dismount the rigid cover. For example, if the rigid cover is screwed, the screw should be turned in the reverse direction when the rigid cover is dismounted,and this operation should be performed with both hands, Accordingly, the operation efficiency is low, and since both hands are used for the dismounting operation, the dirty endoscope should be put at a certain place and the problem of contamination arises.

It is a primary object of the present invention to protect the intermediate conduit portion of the endoscope from contamination during use, and turns next use immediately after the use into posibility.

Another object of the present invention is to provide an endoscope cover, in which, the rigid cover is prevented from being taken off or getting out of position, and there can be attained an effect of ensuring complete covering.

Still another object of the present invention is to provide an endoscope cover, which can be taken off from the endoscope by one touch, and the removal of the used endoscope cover can be accomplished without contamination of the endoscope.

Summary of the Invention

In accordance with the present device, the foregoing problems are solved by an endoscope cover comprising a cylindrical rigid cover composed of an elastic material, which is fitted to an endoscope to cover a top end rigid portion of the endoscope, a transparent window arranged on a closed face formed on the top end of the rigid cover to confront at least an observation port and an illuminating port, which are arranged on the top end of the endoscope, and a bag-shaped covering member having a length covering at least an intermediate conduit portion of the endoscope, which is attached to the rear part of the endoscope, wherein the rigid cover is bonded to the top end rigid portion by an adhesive and the covering member is bonded to all or a part of the circumference of the intermediate conduit portion to cover the intermediate conduit portion.

In the above-mentioned structure, if the rigid cover is fitted by bonding using an adhesive to cover the top end rigid portion of the endoscope, headed by the covering member, the transparent window is positioned and fixed to confront the observation port and illuminating port.

The covering member is extended along the intermediate conduit portion and is bonded to all or a part of the intermediate conduit portion by an adhesive to cover the intermediate conduit portion entirely, In this state, the intermediate conduit portion can be inserted into the body cavity and used for the observation. After the use, the rigid cover is pulled by a force, whereby the rigid cover can be separated from the top end rigid portion. Furthermore, by injecting air under pressure into the covering member, the covering member is peeled from the intermediate conduit portion, and by pulling the covering member, it is possible to draw out the intermediate conduit portion from the covering member without contamination. By fitting a new endoscope cover to the endoscope, the endoscope can be directly used again.

Brief Description of the Drawings

Fig. 1 is a side view illustrating the state where an endoscope cover is attached to an endoscope.

Fig. 2 is a perspective view illustrating the state of a top end rigid portion and a rigid cover in the endoscope.

Fig. 3 is a sectional view illustrating the state where the endoscope cover is taken off from the endoscope.

Detailed Description of the Preferred Embodiments

An example of the present device will now be described with reference to the accompanying drawings.

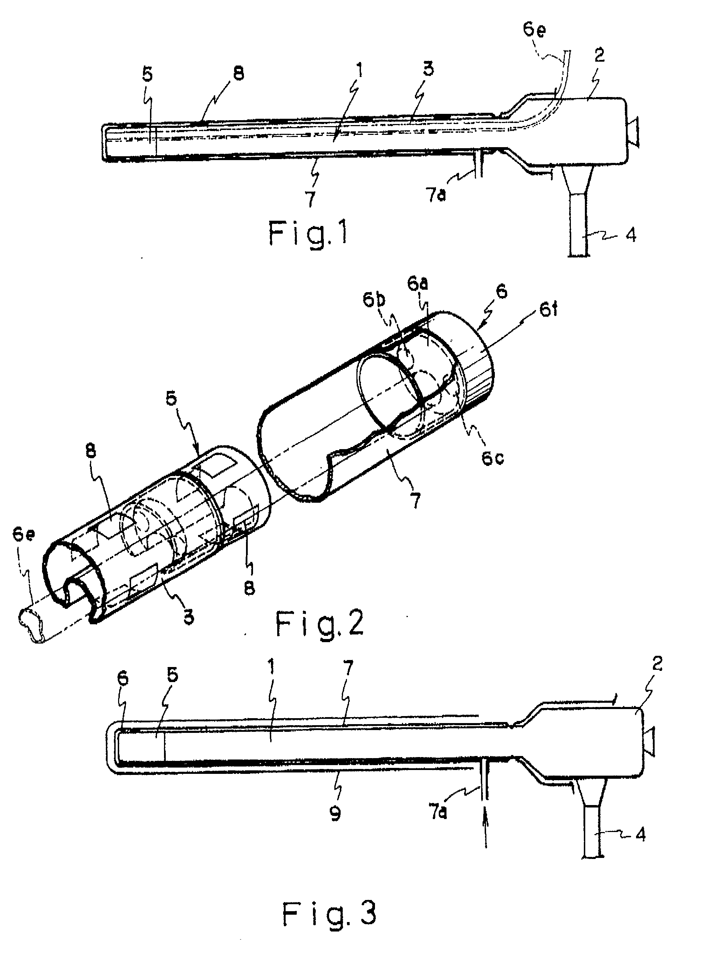

Fig. 1 is a side view illustrating the state where an endoscope cover is attached to an endoscope. Fig. 2 is a perspective view illustrating the state of a top end rigid portion of the endoscope and a rigid cover. Fig. 3 is a sectional view showing the state where the endoscope cover is taken off from the endoscope. In the drawings, reference numeral 1 represents an endoscope comprising a handle 2, an intermediate conduit portion 3 composed of a flexible tube and a guide tube portion 4 connected to a light source.

In general, an image guide, a light guide and an angle-operating wire are disposed in the intermediate conduit portion 3.

The top portion of the intermediate conduit portion 3 forms a bendable angle portion, and a top end rigid portion 5 is attached to the top end of the angle portion.

An observation port connected to an image guide and an illuminating port connected to a light guide are formed on the top end of the top end rigid portion 5.

Reference numeral 5a represents a fitting groove in which a tube having both the ends opened, such as a forceps guide tube continuous from the intermediate conduit portion 3 to the top end rigid portion 5 or an air/water supply tube, is fitted.

Reference numeral 6 represents a rigid cover, which has a cylindrical shape having an inner diameter sufficient to cover the top end rigid portion 5, and transparent windows 6b and 6c are formed on a closed face 6a on the top end of the rigid cover 6 to confront the observation port and illuminating port.

A hood 6f extended to the outer periphery of the top end is formed. If necessary, a hole corresponding to the above-mentioned fitting groove is formed on the closed face 6a and the end portion of the tube 6e having both the ends opened, such as the forceps guide tube, is attached to this hole. Of course, this hole need not be forced in case of an endoscope not using a forceps or the like, and in this case, the above-mentioned fitting groove and the tube having both the ends opened need not be formed.

A soft bag-shaped covering member 7 formed of a rubber or synthetic resin is attached to the outer periphery of the rigid cover 6, and the diameter of the covering member 7 is adjusted to a value almost equal to the outer diameter of the intermediate conduit portion 3 so that the covering member 7 adheres closely to the intermediate conduit portion 3, or adjusted to a value slightly larger than the outer diameter of the intermediate conduit portion 3 so that the covering member 7 loosely covers the intermediate conduit portion 3. In each case, the end portion of the covering member 7 adheres and anchors closely to the intermediate conduit portion 3 at the position of the root of the handle 2 or covers the handle 2 entirely.

In the example having the above-mentioned structure, if the rigid cover 6, headed by the covering member 7, is fitted to cover the top end rigid portion 5 of the endoscope, and the covering member 7 is bonded to all or a part including the front surface by an adhesive 8. It is sufficient if the bonding force and bonding area of the adhesive 8 are such that the rigid cover 6 or the covering member 7 does get out of order during the use. If the bonding force is too strong, there is a risk of reduction of the operation efficiency when the rigid cover 6 or covering member 7 is taken out.

Then, the covering member 7 is extended from the rear part along the intermediate conduit portion 3 and bonded to all or a part of the surface of the intermediate conduit portion 3 to cover the intermediate conduit portion 3 entirely or to cover even the handle 2 as well as the intermediate conduit portion 3. In this state, the intermediate conduit portion 3 is inserted into the body cavity and used for the observation.

The adhesive 8 can be applied by brush coating or spray coating, or a double-coated adhesive tape can be used. Furthermore, there can be adopted a method in which the adhesive 8 is not applied to the top end rigid portion 5 or the intermediate conduit portion 3, but the adhesive 8 is applied to the covering member 7 in advance.

After the observation, the endoscope cover is taken out from the endoscope. Namely, at first, as shown in Fig. 3, the endoscope is put into a transparent bag 9 having a length sufficient to contain the entire endoscope therein or contain at least the entire intermediate conduit portion 3 therein, so that the operation is carried out in the state where the endoscope is grasped through the bag 9.

At first, air is injected under pressure into the covering member 7 from the air-injecting opening 7a to separate the endoscope cover from the endoscope, and by pulling the rigid cover 6 by a force, the rigid cover can be separated from the top end rigid portion 5 and the intermediate conduit portion 3 can be drawn out from the endoscope cover by pulling it together with the bag 9. The endoscope cover is thrown away together with the bag 9, and the endoscope is not contaminated.

Accordingly, by attaching a new endoscope cover to the endoscope, the endoscope can be immediately used again.

As is apparent from the foregoing detailed description, according to the present device, there is provided an endoscope cover comprising a cylindrical rigid cover composed of an elastic material, which is fitted to an endoscope to cover a top end rigid portion of the endoscope, a transparent window arranged on a closed face formed on the top end of the rigid cover to confront at least an observation port and an illuminating port, which are arranged on the top end of the endoscope, and a bag-shaped covering member having a length covering at least an intermediate conduit portion of the endoscope, which is attached to the rear part of the endoscope, wherein the rigid cover is bonded to the top end rigid portion by an adhesive and the covering member is bonded to all or a part of the circumference of the intermediate conduit portion to cover the intermediate conduit portion. In this structure, even if the endoscope is used, for example, in the body cavity, since the intermediate conduit portion, that is, the inserting portion, is covered with the covering member, the endoscope is not contaminated, and only by exchanging the endoscope cover with a new endoscope cover after the use, the endoscope can be immediately used again.

Moreover, since the rigid cover can be fixed to the top end rigid portion and the covering member is bonded to the intermediate conduit portion, the rigid cover is prevented from being taken off or getting out of position, and there can be attained an effect of ensuring complete covering.

Moreover, since the endoscope cover can be taken off from the endoscope by one touch, the removal of the used endoscope cover can be accomplished without contamination of the endoscope. This is another effect attained by the present device.

## Claims

1. An endoscope cover comprising a cylindrical rigid cover composed of an elastic material, which is fitted to an endoscope to cover a top end rigid portion of the endoscope, a transparent window arranged on a closed face formed on the top end of the rigid cover to confront at least an observation port and an illuminating port, which are arranged on the top end of the endoscope, and a bag-shaped covering member having a length covering at least an intermediate conduit portion of the endoscope, which is attached to the rear part of the endoscope, wherein the rigid cover is bonded to the top end rigid portion by an adhesive and the covering member is bonded to all or a part of the circumference of the intermediate conduit portion to cover the intermediate conduit portion.

2. An endoscope cover as set forth in claim 1, wherein an air-injecting opening is formed at the rear end of the covering member.

Fig.1

Fig.2

Fig.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | GB-A-2 218 636   (RHYS AP DELWYN PHILLIPS) <br> * page 2, line 15 - page 3, line 24 * * page 5, lines 16 - 33; figures * | 1 | A 61 B <br> 1/00 |
| P,X | US-A-4 922 914   (E.O. SEGAL & W.R.SEGAL) <br> * column 3, lines 28 - 59 * * column 5, lines 6 - 50; figures 2, 5 * | 1 | |
| D,A | EP-A-0 184 778   (F.E. SILVERSTEIN & E.A. OPIE) <br> * page 10, line 15 - page 12, line 25 * | 1,2 | |
| A | US-A-4 878 485   (E.L. ADAIR) <br> * column 3, line 64 - column 5, line 44 * | 1,2 | |
| A | DE-A-3 233 564   (FUJI OPTICAL CO., LTD.) <br> * page 7, lines 5 - 26; figures 1-3 * * page 9, line 8 - page 10, line 27 * | 1,2 | |
| | | | TECHNICAL FIELDS SEARCHED (Int. Cl.5) <br><br> A 61 B |

The present search report has been drawn up for all claims

| Place of search | Date of completion of search | Examiner |
|---|---|---|
| The Hague | 10 May 91 | RIEB K.D. |